# EUROPÄISCHE PATENTSCHRIFT

(11) **EP 4 388 920 B1**
(45) Veröffentlichungstag und Bekanntmachung des Hinweises auf die Patenterteilung: **29.01.2025**
(21) Anmeldenummer: 22216544.1
(22) Anmeldetag: 23.12.2022
(51) Int. Cl.: A45C 5/00, A45C 5/03, A45C 13/26, A61B 50/31, B65D 25/28, A45C 7/00, A45C 13/18, A45C 13/28, A45C 13/00

(54) **NOTFALLKOFFER SOWIE VERWENDUNG EINES (NOTFALL-)KOFFERS**
EMERGENCY CASE AND USE OF A (EMERGENCY) CASE
VALISE D'URGENCE ET UTILISATION D'UNE VALISE D'URGENCE

(43) Veröffentlichungstag der Anmeldung: 26.06.2024
(73) Patentinhaber: W. Söhngen GmbH, 65232 Taunusstein (DE)
(72) Erfinder: MAY, Melanie, 65193 Wiesbaden (DE); ARNOLD, Pascal, 66265 Heusweiler (DE)
(74) Vertreter: Hoffmann, Jürgen

(56) Entgegenhaltungen:
- CN-U- 214 550 771
- DE-A1- 4 207 877
- DE-U1- 29 720 749
- FR-A5- 2 038 850
- US-A1- 2014 311 845

## Beschreibung

Die Erfindung betrifft einen Notfallkoffer.

Unter Notfallkoffer wird hier vorliegend insbesondere verstanden ein Koffer, der befüllt ist mit zumindest einem aus: Pflaster und Verbandsmaterial, Einmalhandschuhen, Erste-Hilfe-Scheren und Pinzetten, Hygienemundschutz, Hygienekleidung, Notduschen und Augenspülung, Desinfektionsmittel und Spender dazu, Defibrillatoren und Zubehör, sowie Beatmungsmasken.

Die Erfindung betrifft insbesondere die Ausgestaltung eines Griffs an dem Notfallkoffer.

Ein Notfallkoffer mit zwei Griffteilen an zwei sich zueinander öffnenden Hälften ist aus der CN 0214550771 U bekannt.

Ferner ist aus der Notfallmedizin ein teilbarer Koffer gemäß der CN 208877606 U bekannt, bei dem an jedem der Bauteile ein Griffteil angeordnet ist, wobei sich die beiden Griffteile ergänzen, wenn die Teile des Koffers zusammengefügt sind.

US2014/311845 beschreibt auch einen Koffer.

Es ist Aufgabe der Erfindung, einen verbesserten Notfallkoffer zu schaffen und entsprechende Nutzungen zu ermöglichen.

Die Aufgabe wird durch einen Notfallkoffer mit den Merkmalen nach Anspruch 1 und durch die Verwendung eines Koffers gemäß Anspruch 20 bzw. eines Notfallkoffers gemäß Anspruch 21 gelöst.

Der erfindungsgemäße Notfallkoffer umfasst zumindest eine Kofferschale, wobei an der Kofferschale ein Griff ausgebildet ist. Dieser Griff ist mit zwei Ansatzenden jeweils an einer oberen Außenecke der Kofferschale angeordnet. Der Begriff der "oberen Außenecke" bezieht sich auf eine stehende Position des Notfallkoffers. Ausgehend von jeder der oberen Außenecken erstreckt sich der Griff in einem ersten Abschnitt vertikal nach oben. (Da es zwei Außenecken gibt, gibt es zwei erste Abschnitte). In einem auf den ersten Abschnitt vorzugsweise folgenden zweiten Abschnitt erstreckt sich der Griff zu der den (Außen-)Ecken abgewandten Innenlängskante der Kofferschale hin. (Da sich der erste Abschnitt vertikal nach oben erstreckt, ist hierunter zu verstehen, dass sich der zweite Abschnitt ebenfalls oberhalb der Kofferschalenoberseite erstreckt, wobei sich das Erstrecken zur Innenlängskante der Kofferschale hin auch so formulieren lässt, dass in Draufsicht der zweite Abschnitt die Innenlängskante erreicht). Ein die beiden zweiten Abschnitte verbindender dritter Abschnitt des Griffs erstreckt sich des Weiteren entlang der Innenlängskante. Mit anderen Worten erstreckt sich der Griff ausgehend von einer ersten oberen Außenecke in einem ersten Abschnitt vertikal nach oben, dann zur Innenlängskante im zweiten Abschnitt, dann entlang der Innenlängskante im dritten Abschnitt, dann wieder zurück im zweiten Abschnitt zur Außenseite der Kofferschale hin, und dann vertikal nach unten zur zweiten Außenecke hin.

Ein solcher Notfallkoffer hat den Vorteil, dass durch die Befestigung des Griffs an den Außenecken einerseits und des Vorhandenseins des dritten Abschnitts entlang der Innenlängskante zum einen der Transport erleichtert ist, weil der Griff selbst für ein Austarieren des am dritten Abschnitt mit der Hand gehaltenen Koffers sorgt.

Zudem ist die in Patentanspruch 18 genannte Verwendung ermöglicht: Ausgehend von der Situation, dass der Notfallkoffer auf einer Außenseite der Kofferschale liegt bzw. mit einer Außenseite der Kofferschale auf dem Boden oder einer sonstigen Unterlage aufliegt, kann die Bedienperson den Koffer mit der Hand an dem dritten Abschnitt ergreifen und zu sich hin ziehen. Diese Verwendung ist bei herkömmlichen Notfallkoffern mit etwa abgerundeten Griffen keineswegs so möglich.

Notfallkoffer müssen sich leicht tragen lassen und schnell zur Hand sein. Dies gilt für den erfindungsgemäßen Notfallkoffer: Er kann auch dann, wenn ein behandelnder Arzt vor einem Patienten steht oder kniet, umstandslos beigezogen werden.

Der Arzt oder sonstiges Krankenwagenpersonal oder dergleichen kann den Koffer insbesondere neben einem Patienten auf dem Boden abstellen, sich dann zum Patienten hinknien und durch Ausstrecken des Arms den Koffer zu sich heranziehen. Dadurch kann wertvolle Zeit bei der Behandlung des Patienten gewonnen werden.

Gemäß einer bevorzugten Ausführungsform der Erfindung ist der Übergang zwischen dem ersten Abschnitt und dem zweiten Abschnitt abgerundet, damit es keine störenden Ecken gibt. Alternativ oder zusätzlich ist der Übergang zwischen dem zweiten Abschnitt und dem dritten Abschnitt abgerundet, damit es keine störenden Ecken gibt.

Der zweite Abschnitt muss nicht unmittelbar von der Stelle, an der der erste Abschnitt endet, zur Innenlängskante hin verlaufen. Vielmehr kann es vorteilhaft sein, wenn der zweite Abschnitt des Griffs mit einem Teilabschnitt (also partiell) zunächst noch parallel zu einer Außenlängskante der Kofferschale verläuft und sich erst in einem weiteren Teilabschnitt geradlinig zu der Innenlängskante hin erstreckt, wobei der Teilabschnitt mit geradliniger Erstreckung nicht notwendigerweise senkrecht zu der Außenlängskante stehen muss, sondern in einem geeigneten Winkel von beispielsweise 15 bis 30 Grad stehen kann. Dadurch gewinnt der Koffer nicht nur ein gefälliges Aussehen, sondern funktionell ist das Ergreifen von oben erleichtert, und zugleich ist auch ein Gleichgewichtsabgleich beim Halten des Koffers gewährleistet.

Der dritte Abschnitt kann sich mittig entlang der Innenlängskante erstrecken. Naturgemäß ist dies eine besonders stabile Ausführungsform, wenn der dritte Abschnitt, der gerne von der Bedienperson ergriffen wird, mittig verläuft.

Weiter bevorzugt ist dann jeder Griff auch symmetrisch ausgebildet, d.h. die ersten Abschnitte und die zweiten Abschnitte sind zueinander spiegelbildlich, der dritte Abschnitt verbindet diese. Die Spiegelebene schneidet den dritten Abschnitt in zwei Hälften.

Der Notfallkoffer kann im Prinzip aus nur einer einzigen Kofferschale bestehen. Er kann jedoch andererseits auch zwei zueinander komplementäre Kofferschalen aufweisen. Dies kann den Vorteil haben, dass der Koffer leicht, insbesondere symmetrisch, aufklappbar gestaltet sein kann.

Wenn an beiden Kofferschalen je ein Griff vorgesehen ist, wobei vorzugsweise die Griffe zueinander symmetrisch sind, also spiegelgleich ausgestaltet sind, lässt sich der gesamte Notfallkoffer besonders stabil tragen. Dies gilt in besonderem Maße, wenn die dritten Abschnitte der Griffe einander berühren.

Vorzugsweise ist weiterhin vorgesehen, dass am Ende jedes Griffs eine Stapelzentriereinrichtung ausgebildet ist. Unter "Stapelzentriereinrichtung" wird vorliegend verstanden, dass mehrere gleichartige Notfallkoffer oder Kofferschalen zueinander komplementäre Stapelzentriereinrichtungen aufweisen können, so dass durch Formschluss, wenn nicht gar sogar Rastschluss, eine stabile Stapelung ermöglicht ist.

Die Stapelzentriereinrichtungen am Ende jedes Griffs, also an den Außenecken, entsprechen naturgemäß den Stapelzentriereinrichtungen eines zweiten Notfallkoffers, dort auch an den Außenecken. Auch an den unteren Außenecken der Kofferschale können Stapelzentriereinrichtungen vorgesehen sein. Dann kann es unerheblich sein, wie herum die Notfallkoffer ausgerichtet sind, die gestapelt werden.

Bei einer anderen Ausführungsform des Notfallkoffers mit zwei zueinander komplementären Kofferschalen ist nur an einer der beiden Kofferschalen ein Griff ausgebildet, wobei an den Außenecken der Kofferschale ohne Griff jeweils eine Blindkappe vorgesehen ist. Auf diese Weise lässt sich eine Kofferschale wahlweise mit Griff oder mit Blindkappe versehen, z.B. durch geeignete Steckverbindung.

Auch an den Blindkappen kann jeweils eine Stapelzentriereinrichtung ausgebildet sein, die dann eben mit den Stapelzentriereinrichtungen anderer Blindkappen oder an den Enden der Griffe zueinander komplementär ist.

Vorzugsweise sind die Stapelzentriereinrichtungen einer Kofferschale an den oberen Außenecken zueinander komplementär (auf Umschlag). Dann lassen sich gleichartige Kofferschalen gut aufeinanderstapeln.

Vorzugsweise ist jede Kofferschale Teil einer Notfallkofferhälfte, die mit einer anderen Notfallkofferhälfte lösbar verbunden ist. Durch die lösbare Verbindbarkeit lässt sich der Notfallkoffer in zwei Teile auftrennen. Dies ist insbesondere dann vorteilhaft, wenn beide Notfallkofferhälften, also beide Kofferschalen, über einen Griff verfügen.

Vorzugsweise ist vorgesehen, dass jede Kofferschale (im vorherigen Beispiel jede Notfallkofferhälfte) über einen Innendeckel verfügt. Dies ermöglicht es, eine Kofferschale alleine zu tragen, und zwar an dem Griff, wozu die spezielle Ausgestaltung des Griffs mit dem dritten Abschnitt oberhalb der Innenlängskante für Stabilisierung sorgt.

Auch an jedem Innendeckel können Stapelzentriereinrichtungen vorgesehen sein, so dass die Kofferschalen einzeln stapelbar sind.

Gemäß einer weiteren bevorzugten Ausführungsform kann vorgesehen sein, dass der Notfallkoffer fluoresziert. Auf diese Weise ist er bei Nacht gut sichtbar, sodass Zeit bei der Bedienung eingespart werden kann.

Etwa kann der Notfallkoffer eine zumindest partiell fluoreszierend ausgebildete Bewandung haben. Das ist bei Verwendung von Kunststoff erzielbar, indem etwa dem Kunststoffgranulat vor einem Spritzgießen oder sonstiger Formgebung ein fluoreszierender Stoff beigefügt wird. Es kann sich beispielsweise um einen photolumineszierenden Stoff, insbesondere einen photolumineszierenden Stoff mit langer Nachleuchtzeit handeln, mit dem beispielsweise eine Leuchtdichte von 55/8 mcd/qm gemäß DIN 67510 erzielbar ist. Beispielsweise kann es sich bei dem fluoreszierenden Stoff um den aus EP000000853112A1 an sich bekannten Stoff handeln; insbesondere kann der Stoff fluoreszierende Partikel beinhalten, insbesondere mit upconversion oder downconversion. In diesem Fall leuchten die Partikel auch dann, wenn über längere Zeit eine Anregung mit dem Umgebungslicht unterbleibt, beispielsweise im Falle eines Stromausfalls. Reststrahlung in einem anderen Frequenzbereich wird dann durch upconversion oder downconversion von diesen Partikeln im sichtbaren Bereich abgegeben, sodass der Notfallkoffer auch in diesem Fall gut sichtbar bleibt.

Alternativ oder zusätzlich können die Bewandungen des Notfallkoffers fluoreszierend bedruckt und/ oder mit fluoreszierender Folie beklebt werden.

Weiterhin ist es möglich, den Griff fluoreszieren zu lassen. Es kann also ein zumindest partiell fluoreszierend ausgebildeter und/ oder fluoreszierend bedruckter und/ oder mit fluoreszierender Folie beklebter Griff bereitgestellt werden. Dies lässt sich insbesondere an einem Griffeinsatz verwirklichen. Dann muss der restliche Griff nicht, und ggf. auch nicht der restliche Notfallkoffer, fluoreszierend ausgebildet sein, was die Herstellung preisgünstiger macht.

Dies kann beinhalten, dass der Griff alleine leuchtet. Dann ist der Griff besonders gut sichtbar, was bei Dunkelheit ein Ergreifen vor dem Heranziehen erleichtert. Dieser Effekt kann bei fluoreszierender Bewandung ebenfalls erzielt werden, indem der Griff heller fluoresziert als die Bewandung. Das Material für den Griff kann eine höheren Anteil an fluoreszierenden Partikeln aufweisen als der Notfallkoffer im übrigen.

Nachfolgend wird die Erfindung unter Bezug auf die Zeichnung, näher beschrieben, in der
- Fig. 1: eine perspektivische Ansicht einer ersten Ausführungsform des erfindungsgemäßen Notfallkoffers zeigt;
- Fig. 2: eine Vorderansicht des Notfallkoffers aus Fig. 1 zeigt;
- Fig. 3: eine Draufsicht des Notfallkoffers aus Fig. 1 zeigt;
- Fig. 4: eine Unteransicht des Notfallkoffers aus Fig. 1 zeigt;
- Fig. 5: eine Seitenansicht des Notfallkoffers aus Fig. 1 zeigt;
- Fig. 6a: den Notfallkoffer aus Fig. 1 zeigt, wenn er geöffnet ist,
- Fig. 6b: dies weiter veranschaulicht, wenn die beiden Innendeckel geöffnet sind,
- Fig. 6c: dies weiter veranschaulicht, wenn die beiden Innendeckel zur einen Seite herumgeklappt sind;
- Fig. 7a: eine zweite Ausführungsform des erfindungsgemäßen Notfallkoffers zeigt, in perspektivischer Ansicht von einer ersten Seite, und
- Fig. 7b: diesen von einer zweiten Seite zeigt; und
- Fig. 8: eine Vorderansicht des Notfallkoffers aus Fig. 7a zeigt;
- Fig. 9a und Fig. 9b: zwei verschiedene Seitenansichten des Notfallkoffers aus Fig 7a zeigt;
- Fig. 10: eine Draufsicht des Notfallkoffers aus Fig. 7a zeigt; und
- die Fig. 11a bis 11c: den Notfallkoffer aus Fig. 7a in Darstellungen entsprechend den Figuren 6a bis 6c veranschaulicht;
- Fig. 12a: eine Notfallkofferhälfte als Notfallkoffer gemäß einer dritten Ausführungsform der Erfindung veranschaulicht in perspektivischer Ansicht von einer ersten Seite, und
- Fig. 12b: diesen von einer zweiten Seite veranschaulicht;
- Fig. 13: ein Stapelzentrierelement an der linken oberen Außenecke des Notfallkoffers aus Fig, 7;
- Fig, 14: einen Stapel von mehreren Notfallkoffern;
- Fig, 15a: einen Ausschnitt aus Fig, 1 mit dem Griff; und
- Fig, 15b: einen Ausschnitt aus Fig, 6a mit dem Griff zeigt.

Ein im Ganzen mit 100 bezeichneter Notfallkoffer umfasst zwei Kofferschalen 10, die vorliegend gleichartig sind und im Falle des Notfallkoffers 100 jeweils gleichartige Griffe 12 tragen. Die Kofferschalen 10 mit den Griffen 12 bilden zusammen eine Notfallkofferhälfte, wobei im Falle des Notfallkoffers 100 zwei Notfallkofferhälften exakt zueinander komplementär sein können.

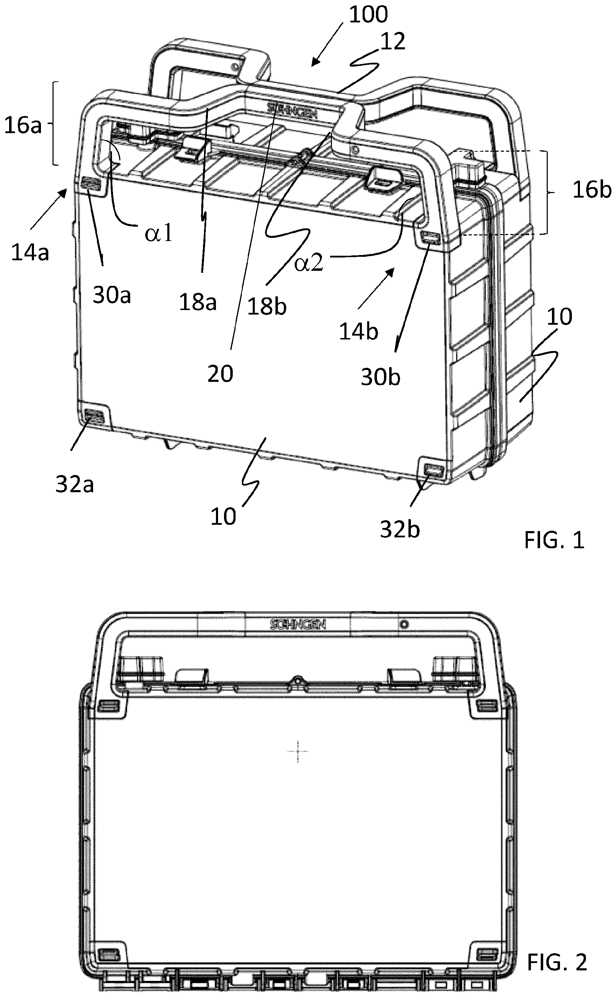

Die Griffe sind an den oberen Außenecken 14a, 14b jeder Kofferschale 10 befestigt, d.h. ein erstes Ende des Griffs 12 ist an der ersten oberen Außenecke 14a eingesteckt und ein zweites Ende des Griffs 12 an der anderen oberen Außenecke 14b der Kofferschale 10 eingesteckt. In einem ersten Abschnitt ausgehend von der jeweiligen oberen Außenecke erstreckt sich der Griff 12 vertikal nach oben, vorliegend sind dies die in Fig. 1 zu sehenden Abschnitte 16a (erster Abschnitt an dem ersten Griffende) und 16b (erster Abschnitt am zweiten Griffende). Unter "vertikal nach oben" wird verstanden, dass der Anstellwinkel α1 am ersten Griffende und der Anstellwinkel α2 am zweiten Griffende jeweils zwischen 70 Grad und 110 Grad liegt, vorzugsweise kleiner als 90° ist. Vorliegend sind beide Anstellwinkel α1 und α2 vorzugsweise gleich und haben hierzu beispielsweise einen Wert von ca. 75 Grad.

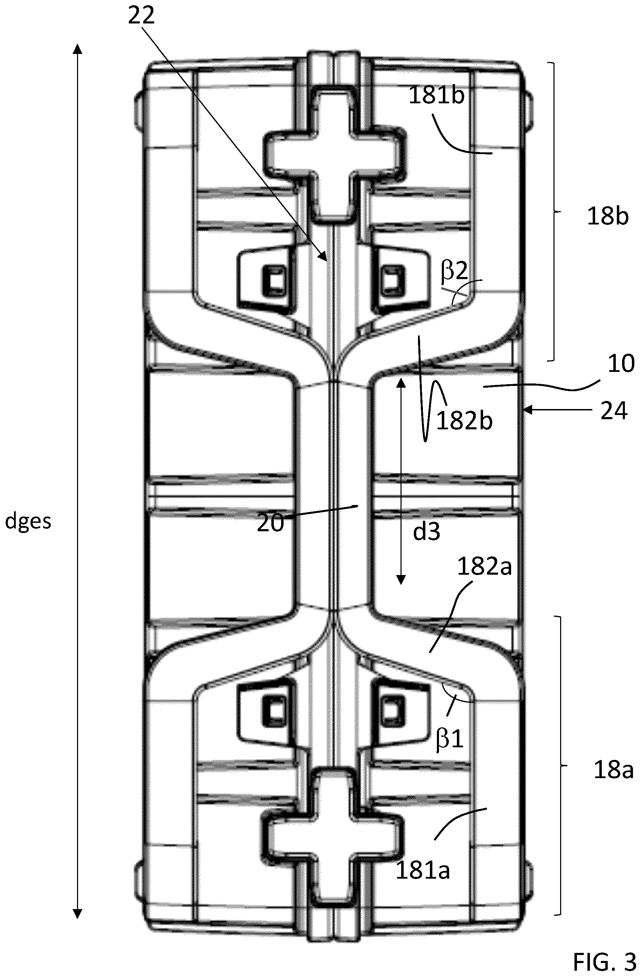

Aus der Draufsicht gemäß Fig. 3 ist erkennbar, wie sich der Griff 12 fortsetzt: In einem zweiten Abschnitt 18a bzw. 18b erstreckt sich jeder Griff zu der Innenlängskante 22 der Kofferschale hin. Vorliegend beinhaltet der zweite Abschnitt zwei Teilabschnitte, nämlich der Abschnitt 18a den Abschnitt 181a, der noch parallel zur Außenlängskante 24 der Kofferschale 110 verläuft, und den Abschnitt 182a, der von der Außenlängskante 24 hin zur Innenlängskante 22 verläuft. Der Winkel β1 der Anstellung des zweiten Teilabschnitts 182a gegenüber dem ersten Teilabschnitt 181a beträgt zwischen 10 und 20 Grad, vorliegend ca. 15 Grad. Der zweite Abschnitt 18b ist in analoger Weise in die Teilabschnitte 181b und 182b unterteilt, bei denen der Winkel β2 vorzugsweise ebenfalls zwischen 10 und 20 Grad liegt und vorzugsweise denselben Wert wie der Winkel β1 hat.

Am Ende des zweiten Abschnitts 18b, insbesondere am Ende dessen zweiten Teilabschnitts 182b im Bereich der Innenlängskante 22 erstreckt sich der dritte Abschnitt 20 des Griffs 12. Dieser mittige Abschnitt erstreckt sich insbesondere parallel zur Innenlängskante 22, und bei dem Notfallkoffer 100 berühren sich der dritte Abschnitt 20 der ersten Notfallkofferhälfte an der ersten Kofferschale 10 und der dritte Abschnitt 20 der entsprechenden zweiten Notfallkofferhälfte unmittelbar oberhalb der Innenlängskante 22. Die Länge d3 des dritten Abschnitts 20 beträgt nahezu ein Drittel der Gesamtlänge dges des gesamten Koffers und damit des gesamten Griffs 12, wobei die Abschnitte 18b und 18a das andere Drittel ergänzen, bzw. geringfügig länger als das Drittel sind. Durch diese Abmessungen ist für eine Austarierung des Koffers gesorgt, wenn die Bedienperson den Koffer an den beiden dritten Abschnitten 20 umgreift.

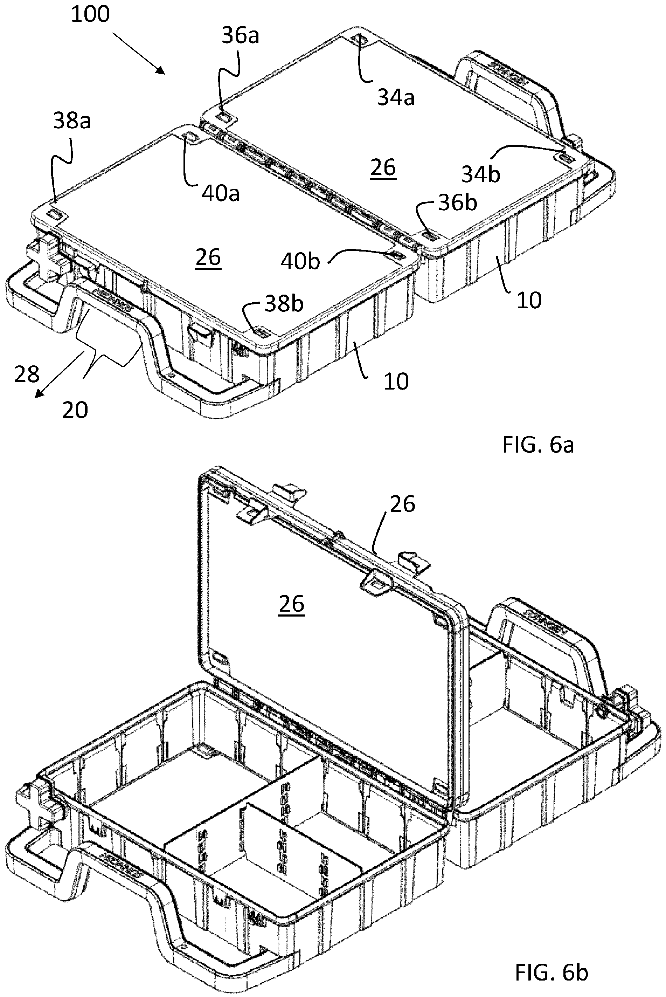

Die Koffer lassen sich gemäß Fig. 6a aufklappen, wodurch sichtbar wird, dass es einen vorzugsweisen opaken Innendeckel 26 an der Kofferschale 10 gibt. Der aufgeklappte Notfallkoffer lässt sich an dem beim Liegen nach oben hochstehenden dritten Abschnitt optimal ergreifen, so dass für die Bedienung in einer Notfallsituation am Patienten vor Ort ein schnelles Heranziehen des Koffers ermöglicht ist, etwa in die Richtung gemäß dem Pfeil 28 in Fig. 6a.

Gemäß Fig. 6b lassen sich die beiden Innendeckel 26 hochklappen und komplementär zueinander senkrecht hochstellen.

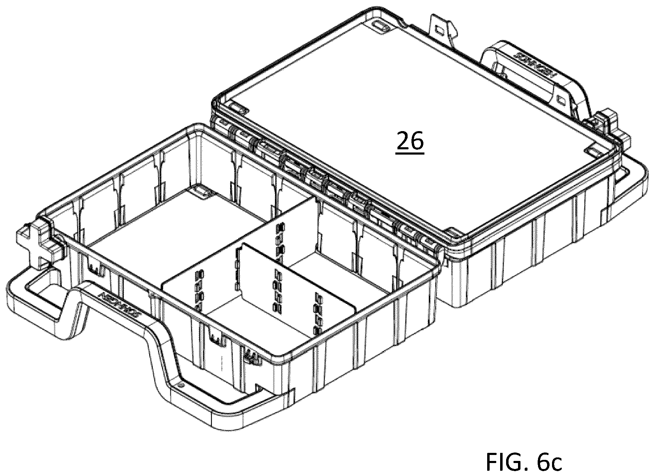

Gemäß Fig. 6c lassen sich die beiden miteinander verrasteten Deckel auch nur zu einer Seite hinklappen, vorliegend der hinteren Seite in Fig. 6c.

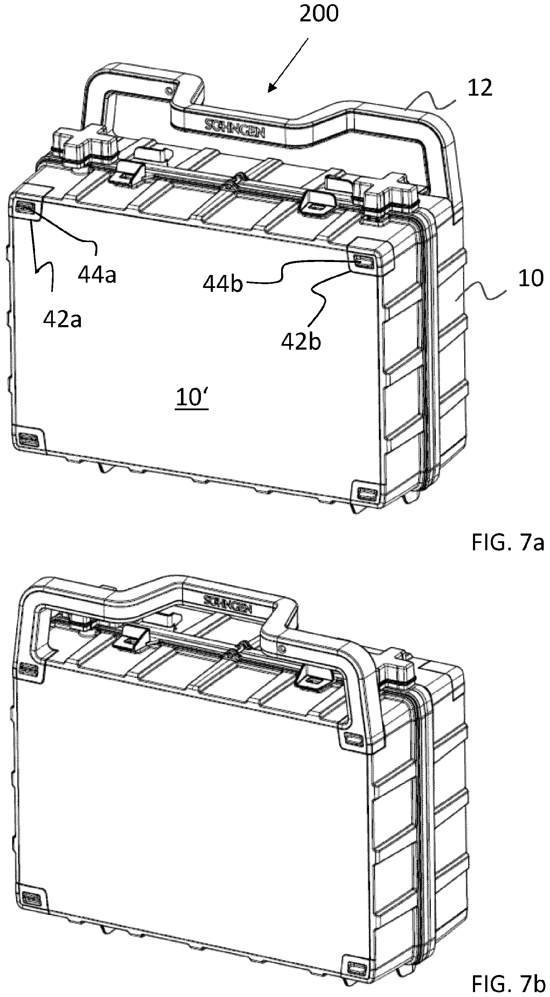

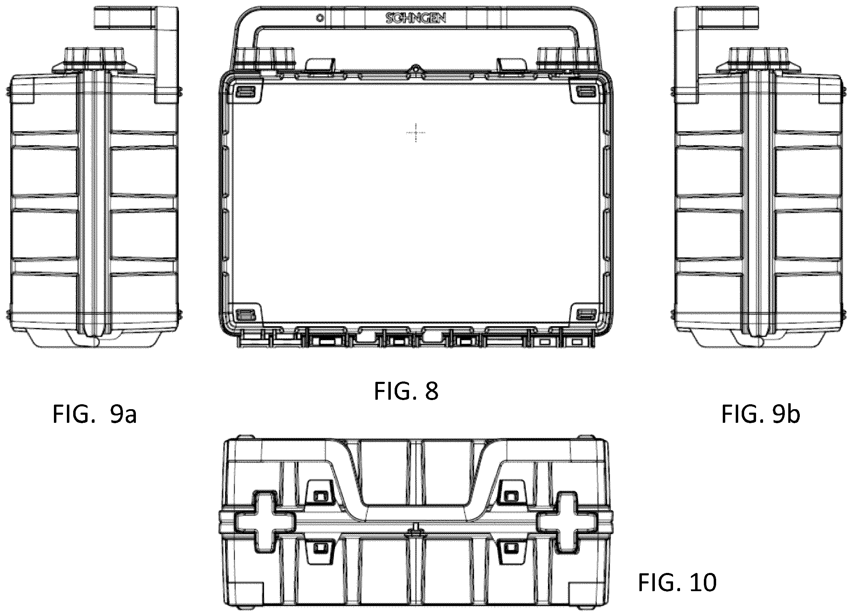

Die nachfolgend erläuterte zweite Ausführungsform 200 des Notfallkoffers gemäß Fig. 7a bis Fig. 11c unterscheidet sich von der bisher beschriebenen Ausführungsform 100 des Notfallkoffers darin, dass an nur einer Kofferschale 10 der Griff 12 ausgebildet ist, wohingegen an der anderen Kofferschale 10' an den oberen Außenecken Blindkappen 42a, 42b eingebracht sind. Die Blindkappen ersetzen den Griff bei der Kofferschale 10'.

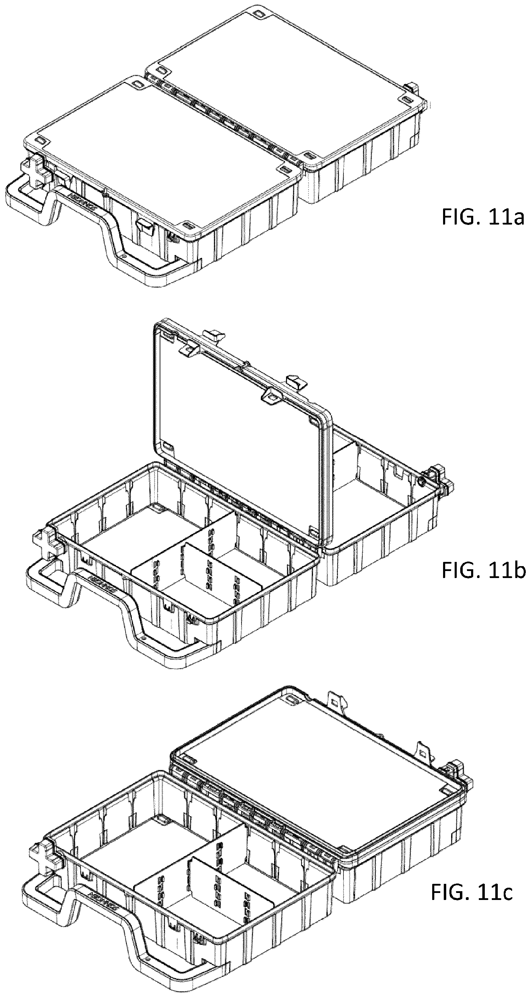

Durch das Entfallen eines Griffs wird der Notfallkoffer 200 leichter als der Notfallkoffer 100. Die Tragbarkeit ist durch die Form des Griffes allerdings nach wie vor sehr erleichtert. Die Figuren 11a bis 11c entsprechen den Figuren 6a bis 6c.

Damit die Notfallkoffer mit anderen Notfallkoffern (etwa in einem Krankenwagen) geeignet stapelbar sind, gibt es sogenannte Stapelzentriereinrichtungen. Solche Einrichtungen sind in der Regel paarweise zueinander komplementär (auf Umschlag).

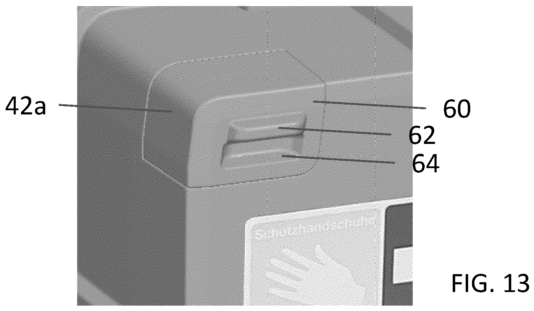

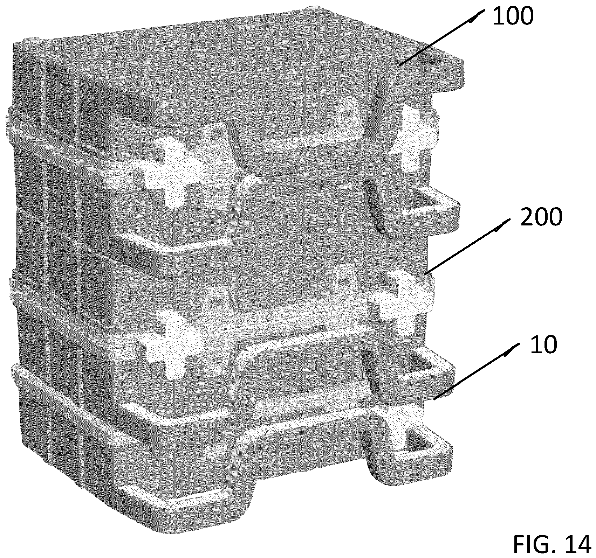

Fig. 13 veranschaulicht eine Stapelzentriereinrichtung an einer linken oberen Außenecke des Notfallkoffers 200, also an der Blindkappe 42a. Die diesbezügliche Beschreibung einer beispielhaften Ausgestaltung gilt für alle Stapelzentriereinrichtungen. Ausgehend von einer glatten, ebenen Oberfläche, die sich an die Oberfläche außerhalb der Blindkappe 42a anschließt, gibt es einen Vorsprung 62, hier mit einer Längserstreckung parallel zur Oberkante der Kofferschale 10. Unterhalb des Vorsprungs 62 gibt es eine Ausnehmung 64, die in der Form komplementär zum Vorsprung 62 ist. Die Rollen von Vorsprung und Ausnehmung sind an den auf Umschlag vorgesehenen Stapelzentriereinrichtungen vertauscht. Etwa an der rechten oberen Außenecke ist dort an der Stapelzentriereinrichtung 42b eine Ausnehmung oben und ein Vorsprung darunter vorgesehen. So passt der Vorsprung einer Kofferschale in die Ausnehmung an einer anderen eines anderen Notfallkoffers.

Im Einzelnen zu den Stapelzentriereinrichtungen: Beim Notfallkoffer 100 sind an den Griffenden an den oberen Außenecken der Kofferschale 10 die Stapelzentriereinrichtungen 30a und 30b vorgesehen, die zueinander auf Umschlag sind. An den unteren Ecken sind ihrerseits Stapelzentriereinrichtungen 32a, 32b ausgebildet, wobei die Stapelzentriereinrichtung 32a mit Stapelzentriereinrichtung 32b komplementär ist. Die Stapelzentriereinrichtung 32a ist ferner mit der Stapelzentriereinrichtung 30a komplementär, die Stapelzentriereinrichtung 32b mit der Stapelzentriereinrichtung 30b komplementär (auf Umschlag).

Damit auch geöffnete Notfallkoffer stapelbar sind, weisen ferner die Innendeckel 26 Stapelzentriereinrichtungen auf, siehe an dem einen Innendeckel die Bezugszeichen 34a, 34b und 36a und 36b. Die dazu komplementären Stapelzentriereinrichtungen 38a und 38b an der anderen Kofferhälfte und 40a, 40b, sind paarweise komplementär zu den Stapelzentriereinrichtungen: 38a zu 34a, 38b zu 34b, 40a zu 36a und 40b zu 36b. Auf diese Weise lassen sich die beiden Innendeckel 26 auch gut miteinander verrasten, wie in Fig. 6b gezeigt.

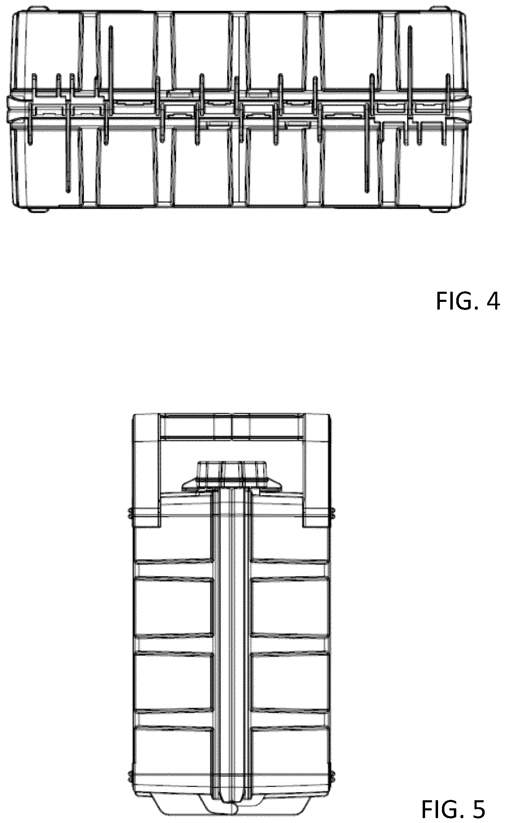

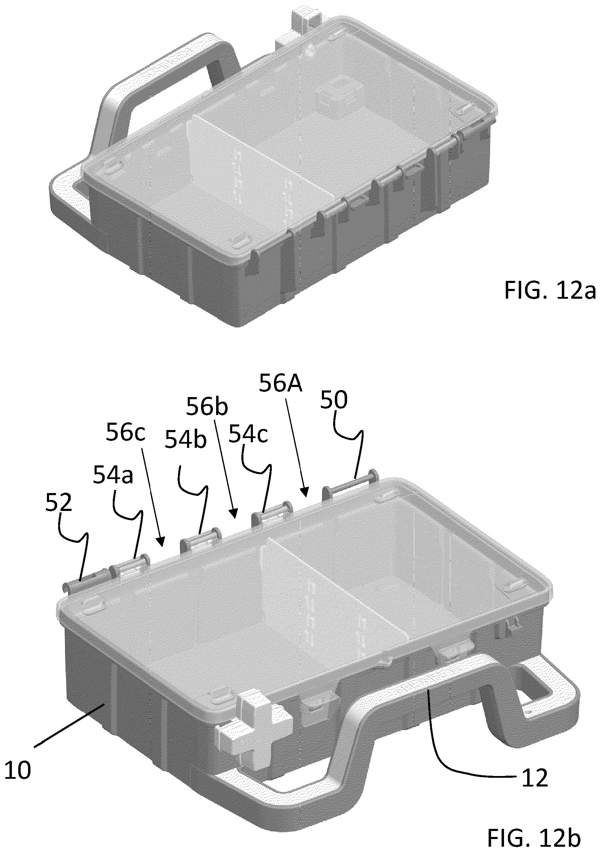

Die beiden Notfallkofferhälften mit den Kofferschalen 10 und dem Griff 12 bei dem Notfallkoffer 100 aus Fig. 1 bis 6c bzw. die entsprechende Kofferhälfte des Notfallkoffers 200 lässt sich auch einzeln verwenden, wie in Fig. 12a und 12b veranschaulicht. Insbesondere sind die beiden Notfallkofferhälften bei den Notfallkoffern 100 und 200 voneinander lösbar. Am unteren Bereich der jeweiligen Notfallkofferhälfte sind zueinander komplementäre Steckvorrichtungen vorgesehen: Eine Stange 50 am unteren Bereich der Notfallkofferhälfte mit der Schale 10 und dem Griff 12 lässt sich in die im Querschnitt C-förmige elastische Steckvorrichtung 52 einer gleich gebauten Notfallkofferhälfte einstecken. Entlang der Achse befinden sich ferner die Einrichtungen 54a, 54b und 54c zur Befestigung des Innendeckels. Die entsprechenden Einrichtungen lassen sich in die Lücken 56a, 56b, 56c der komplementären Notfallkofferhälfte einfügen, so dass eine geschlossene Drehachse gebildet ist.

Fig, 14 zeigt einen Stapel aus a) dem Notfallkoffer wie in Fig 12a, b) dem Notfallkoffer 200 aus Fig, 7a und dem Notfallkoffer 100 aus Fig 1. Zur Stabilität tragen die Stapelzentriereinrichtungen bei.

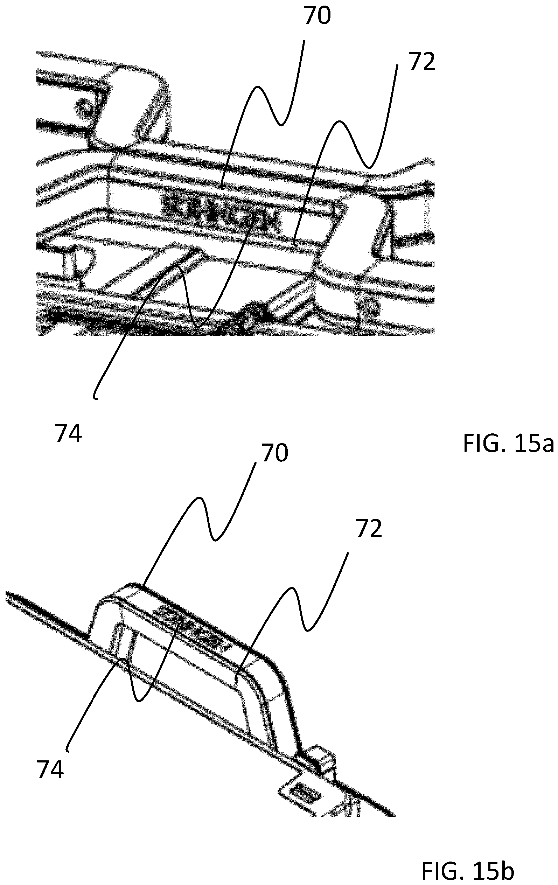

Fig. 15a zeigt einen Ausschnitt aus Fig, 1 mit dem Griff, wie er von außen zu sehen ist. Es ist daraus ersichtlich, dass der Griff einen Rahmen mit oberem Rahmenteil 70 und unterem Rahmenteil 72 aufweist. Zwischen die beiden Rahmenteile 70 und 72 ist ein Einsatzteil 74 des Griffs eingesetzt. Das Einsatzteil 74 kann eingeklebt oder eingepresst sein, ggf. auch mit im Innern des Griffs befindlichen Elementen verclipst sein.

Fig, 15b zeigt einen Ausschnitt aus Fig. 6a mit dem Griff, der darin mit seiner Innenseite sichtbar ist. Auch hier ist zwischen den Rahmenteilen 70 und 72 das Einsatzteil 74 zu sehen. Es kann sich um dasselbe Einsatzteil handeln wie bei Fig. 15a. Alternativ können zwei Einsatzteile vorgesehen sein (nicht dargestellt).

Das Einsatzteil 74 kann fluoreszierend ausgebildet sein, beispielweise durch Beimengung fluoreszierender Partikeln, wie sie von der Fa. American Permalight Corp. unter dem Markennamen PERMALIGHT^{®} vertrieben werden. Das Einsatzteil trägt im Beispiel zudem ein Logo. Dadurch ist der Griff auch bei Nacht gut sichtbar, zum Beispiel für den vor dem Patienten knienden Arzt, der ihn beiziehen möchte.

Der Notfallkoffer kann im Übrigen in anderer Farbe als das Einsatzteil ausgebildet sein. Er kann auch seinerseits fluoreszierend sein, vorzugsweise schwächer fluoreszierend als das Einsatzteil des Griffs. Am einfachsten lässt sich eine fluoreszierende Folie aufbringen, dann kann ein strukturiertes Muster zum Leuchten gebracht werden.

### Bezugszeichenliste

- 10: Kofferschale
- 10': Kofferschale
- 12: Griff
- 14a: obere Außenecke
- 14b: obere Außenecke
- 16a: erster Abschnitt
- 16b: erster Abschnitt
- 18a: zweiter Abschnitt
- 18b: zweiter Abschnitt
- 20: dritter Abschnitt
- 22: Innenlängskante
- 24: Außenlängskante
- 26: Innendeckel
- 28: Pfeil
- 30a: Stapelzentriereinrichtung
- 30b: Stapelzentriereinrichtung
- 32a: Stapelzentriereinrichtung
- 32b: Stapelzentriereinrichtung
- 34a: Stapelzentriereinrichtung
- 34b: Stapelzentriereinrichtung
- 36a: Stapelzentriereinrichtung
- 36b: Stapelzentriereinrichtung
- 38a: Stapelzentriereinrichtung
- 38b: Stapelzentriereinrichtung
- 40a: Stapelzentriereinrichtung
- 40b: Stapelzentriereinrichtung
- 42a: Blindkappe
- 42b: Blindkappe
- 44a: Stapelzentriereinrichtung
- 44b: Stapelzentriereinrichtung
- 50: Stange
- 52: Steckvorrichtung
- 54a: Einrichtung
- 54b: Einrichtung
- 54c: Einrichtung
- 56a: Lücke
- 56b: Lücke
- 56c: Lücke
- 60: ebene Fläche
- 62: Vorsprung
- 64: Ausnehmung
- 70: oberes Rahmenteil des Griffs 12
- 72: unteres Rahmenteil des Griffs 12
- 74: Einsatzteil des Griffs 12100 Notfallkoffer
- 110: Kofferschale
- 200: Notfallkoffer
- 181a: Teilabschnitt
- 181b: Teilabschnitt
- 182a: Teilabschnitt
- 182b: Teilabschnitt

- α1: Anstellwinkel
- α2: Anstellwinkel
- β1: Winkel
- β2: Winkel
- d3: Länge des Abschnitts 20
- dges: Gesamtlänge

## Patentansprüche

1. Notfallkoffer (100, 200), mit zumindest einer Kofferschale (10), wobei an der Kofferschale (10) ein Griff (12) ausgebildet ist, der mit zwei Ansatzenden des Griffs (12) jeweils in einer stehenden Position des Notfallkoffers (100, 200) an einer oberen Außenecke der Kofferschale (10) angeordnet ist, sich in einem ersten Abschnitt (16a, 16b) vertikal nach oben erstreckt und in einem zweiten Abschnitt (18a, 18b) zu der den Ecken abgewandten Innenlängskante (22) der Kofferschale (10) hin erstreckt und wobei sich ein die beiden zweiten Abschnitte (18a, 18b) verbindender dritter Abschnitt (20) des Griffs (12) entlang der Innenlängskante (12) erstreckt.

2. Notfallkoffer (100, 200) nach Anspruch 1, bei dem der Übergang zwischen dem ersten Abschnitt und dem zweiten Abschnitt und/oder der Übergang zwischen dem zweiten Abschnitt und dem dritten Abschnitt abgerundet ist.

3. Notfallkoffer (100, 200) nach Anspruch 1 oder 2, bei dem der zweite Abschnitt (18) des Griffs (12) partiell (181a, 181b) parallel zu einer Außenlängskante der Kofferschale (10) verläuft und sich weiter dann (182a, 182b) geradlinig zu der Innenlängskante hin erstreckt.

4. Notfallkoffer (100, 200) nach einem der Ansprüche 1 bis 3, bei dem sich der dritte Abschnitt (20) mittig entlang der Innenlängskante erstreckt.

5. Notfallkoffer (100, 200) nach Anspruch 4, bei dem jeder Griff (12) symmetrisch ausgebildet ist.

6. Notfallkoffer (100, 200) nach einem der Ansprüche 1 bis 5 mit zwei zueinander komplementären Kofferschalen (10).

7. Notfallkoffer (100) nach Anspruch 6, mit einem Griff (12) an beiden Kofferschalen (10).

8. Notfallkoffer (100) nach Anspruch 7, wobei die dritten Abschnitte (20) der beiden Griffe (12) einander berühren.

9. Notfallkoffer (100) nach Anspruch 7 oder 8, wobei am Ende jedes Griffs (12) eine Stapelzentriereinrichtung (30a, 30b) ausgebildet ist.

10. Notfallkoffer (200) nach Anspruch 6, mit einem Griff (12) an nur einer der beiden Kofferschalen (10), wobei an den Außenecken der Kofferschale (10') ohne Griff jeweils eine Blindkappe (42a, 42b) vorgesehen ist.

11. Notfallkoffer (200) nach Anspruch 9, wobei am Ende des Griffs (12) eine Stapelzentriereinrichtung und wobei an den Blindkappen (42a, 42b) jeweils eine Stapelzentriereinrichtung (44a, 44b) ausgebildet ist.

12. Notfallkoffer (100, 200) nach einem der Ansprüche 9 oder 11, bei dem die beiden Stapelzentriereinrichtungen (30a, 30b, 44a, 44b) einer Kofferschale (10) zueinander komplementär sind.

13. Notfallkoffer (100, 200) nach einem der Ansprüche 1 bis 12, bei dem jede Kofferschale (10) Teil einer Notfallkofferhälfte ist, die mit der anderen Notfallkofferhälfte lösbar verbunden ist.

14. Notfallkoffer (100, 200) nach einem der Ansprüche 1 bis 13, wobei jede Kofferschale (10) über einen Innendeckel (26) verfügt.

15. Notfallkoffer (100, 200) nach Anspruch 14, mit Stapelzentriereinrichtungen (34a, 34b, 36a, 36b, 38a, 38b, 40a, 40b) an jedem Innendeckel (26).

16. Notfallkoffer (100, 200) nach einem der Ansprüche 1 bis 15, mit zumindest partiell fluoreszierend ausgebildeter und/ oder fluoreszierend bedruckter und/ oder fluoreszierend beklebter Bewandung, wobei es sich bei der Fluoreszenz insbesondere um Photolumineszenz, insbesondere mit upconversion und/oder downconversion handelt.

17. Notfallkoffer einem der Ansprüche 1 bis 16, mit zumindest partiell fluoreszierend ausgebildetem und/oder fluoreszierend bedrucktem und/ oder fluoreszierend beklebtem Griff (12), wobei es sich bei der Fluoreszenz insbesondere um Photolumineszenz, insbesondere mit upconversion und/oder downconversion handelt.

18. Notfallkoffer mit den Merkmalen sowohl von Anspruch 16 als auch von Anspruch 17, bei dem der Griff (12) heller fluoresziert als die Bewandung.

19. Notfallkoffer (100, 200) nach einem der Ansprüche 1 bis 18, befüllt mit zumindest einem aus:
Pflaster und Verbandsmaterial;
Einmalhandschuhen;
Erste-Hilfe-Scheren und Pinzetten;
Hygienemundschutz;
Hygienekleidung;
Notduschen und Augenspülung;
Desinfektionsmittel und Spender dazu;
Defibrillatoren und Zubehör;
Beatmungsmasken.

20. Verwendung eines Koffers mit zumindest einer Kofferschale (10), wobei an der Kofferschale (10) ein Griff (12) ausgebildet ist, der mit zwei Ansatzenden des Griffs (12) jeweils in einer stehenden Position des Notfallkoffers (100, 200) an einer oberen Außenecke der Kofferschale (10) angeordnet ist, sich in einem ersten Abschnitt vertikal nach oben erstreckt und in einem zweiten Abschnitt zu der den Ecken abgewandten Innenlängskante der Kofferschale (10) hin erstreckt und wobei sich ein die beiden zweiten Abschnitte verbindender dritter Abschnitt des Griffs (12) entlang der Innenlängskante erstreckt, als Notfallkoffer (100, 200).

21. Verwendung des Notfallkoffers (100, 200) nach einem der Ansprüche 1 bis 19 durch eine medizinische Behandlungsperson in einer medizinischen Notfallsituation, wobei gemäß der Verwendung ausgehend von der Situation, dass der Notfallkoffer (100, 200) auf einer Außenseite der Kofferschale (10) auf dem Boden oder einer Unterlage aufliegt, an dem dritten Abschnitt von der Behandlungsperson mit der Hand ergriffen und zu der Behandlungsperson hin gezogen wird.

## Claims

1. Emergency case (100, 200) having at least one case shell (10), wherein a handle (12) is formed on the case shell (10), which handle, in a standing position of the emergency case (100, 200), is arranged with two attachment ends of the handle (12) at an upper outer corner of the case shell (10), extends vertically upwards in a first section (16a, 16b) and, in a second section (18a, 18b), extends towards the inner longitudinal edge (22) of the case shell (10) facing away from the corners, and wherein a third section (20) of the handle (12), connecting the two second sections (18a, 18b), extends along the inner longitudinal edge (12).

2. Emergency case (100, 200) according to Claim 1, wherein the transition between the first section and the second section and/or the transition between the second section and the third section is rounded.

3. Emergency case (100, 200) according to Claim 1 or 2, wherein the second section (18) of the handle (12) runs partially (181a, 181b) parallel to an outer longitudinal edge of the case shell (10) and then continues (182a, 182b) in a straight line towards the inner longitudinal edge.

4. Emergency case (100, 200) according to one of Claims 1 to 3, wherein the third section (20) extends centrally along the inner longitudinal edge.

5. Emergency case (100, 200) according to Claim 4, wherein each handle (12) is symmetrical.

6. Emergency case (100, 200) according to one of Claims 1 to 5, having two mutually complementary case shells (10).

7. Emergency case (100) according to Claim 6, having a handle (12) on both case shells (10).

8. Emergency case (100) according to Claim 7, wherein the third sections (20) of the two handles (12) touch each other.

9. Emergency case (100) according to Claim 7 or 8, wherein a stack-centring device (30a, 30b) is formed at the end of each handle (12).

10. Emergency case (200) according to Claim 6, having a handle (12) on only one of the two case shells (10), wherein a blind cap (42a, 42b) is provided on the outer corners of the case shell (10') without handle.

11. Emergency case (200) according to Claim 9, wherein a stack-centring device is formed at the end of the handle (12), and wherein a stack-centring device (44a, 44b) is formed respectively on the blind caps (42a, 42b).

12. Emergency case (100, 200) according to either of Claims 9 and 11, wherein the two stack-centring devices (30a, 30b, 44a, 44b) of a case shell (10) are complementary to one another.

13. Emergency case (100, 200) according to one of Claims 1 to 12, wherein each case shell (10) is part of an emergency case half which is detachably connected to the other emergency case half.

14. Emergency case (100, 200) according to one of Claims 1 to 13, wherein each case shell (10) has an inner lid (26).

15. Emergency case (100, 200) according to Claim 14, having stack-centring devices (34a, 34b, 36a, 36b, 38a, 38b, 40a, 40b) on each inner lid (26).

16. Emergency case (100, 200) according to one of Claims 1 to 15, having walls that are at least partially fluorescent and/or have fluorescent print and/or fluorescent adhesive, wherein the fluorescence is in particular photoluminescence, in particular with upconversion and/or downconversion.

17. Emergency case according to one of Claims 1 to 16, having a handle (12) which is at least partially fluorescent and/or has fluorescent print and/or fluorescent adhesive, wherein the fluorescence is in particular photoluminescence, in particular with upconversion and/or downconversion.

18. Emergency case having the features of both Claim 16 and Claim 17, wherein the handle (12) fluoresces more brightly than the walls.

19. Emergency case (100, 200) according to one of Claims 1 to 18, filled with at least one of:
plasters and dressing material;
disposable gloves;
first-aid scissors and tweezers;
hygienic face mask;
hygienic clothing;
emergency showers and eyewash;
disinfectants and dispensers for same;
defibrillators and accessories;
breathing masks.

20. Use of a case having at least one case shell (10), wherein a handle (12) is formed on the case shell (10), which handle, in a standing position of the emergency case (100, 200), is arranged with two attachment ends of the handle (12) at an upper outer corner of the case shell (10), extends vertically upwards in a first section and, in a second section, extends towards the inner longitudinal edge of the case shell (10) facing away from the corners, and wherein a third section of the handle (12), connecting the two second sections, extends along the inner longitudinal edge, as an emergency case (100, 200).

21. Use of the emergency case (100, 200) according to one of Claims 1 to 19 by a medical treatment person in a medical emergency situation, wherein according to the use, starting from the situation in which the emergency case (100, 200) rests with an outer side of the case shell (10) on the ground or on a support surface, it is grasped by hand at the third section by the treatment person and pulled towards the treatment person.

## Revendications

1. Mallette d'urgence (100, 200), avec au moins une coque de mallette (10), une poignée (12) étant réalisée sur la coque de mallette (10), laquelle est agencée avec deux extrémités d'appendice de la poignée (12) respectivement dans une position debout de la mallette d'urgence (100, 200) sur un coin extérieur supérieur de la coque de mallette (10), s'étendant dans une première section (16a, 16b) verticalement vers le haut et s'étendant dans une deuxième section (18a, 18b) vers le bord longitudinal intérieur (22) de la coque de mallette (10) détourné des coins, et une troisième section (20) de la poignée (12) reliant les deux deuxièmes sections (18a, 18b) s'étendant le long du bord longitudinal intérieur (12).

2. Mallette d'urgence (100, 200) selon la revendication 1, dans laquelle la transition entre la première section et la deuxième section et/ou la transition entre la deuxième section et la troisième section est arrondie.

3. Mallette d'urgence (100, 200) selon la revendication 1 ou 2, dans laquelle la deuxième section (18) de la poignée (12) est partiellement (181a, 181b) parallèle à un bord longitudinal extérieur de la coque de mallette (10) et s'étend ensuite (182a, 182b) de manière rectiligne vers le bord longitudinal intérieur.

4. Mallette d'urgence (100, 200) selon l'une quelconque des revendications 1 à 3, dans laquelle la troisième section (20) s'étend de manière centrale le long du bord longitudinal intérieur.

5. Mallette d'urgence (100, 200) selon la revendication 4, dans laquelle chaque poignée (12) est réalisée sous forme symétrique.

6. Mallette d'urgence (100, 200) selon l'une quelconque des revendications 1 à 5, avec deux coques de mallette (10) complémentaires l'une de l'autre.

7. Mallette d'urgence (100) selon la revendication 6, avec une poignée (12) sur les deux coques de mallette (10) .

8. Mallette d'urgence (100) selon la revendication 7, dans laquelle les troisièmes sections (20) des deux poignées (12) sont en contact l'une avec l'autre.

9. Mallette d'urgence (100) selon la revendication 7 ou 8, dans laquelle un dispositif de centrage d'empilement (30a, 30b) est réalisé à l'extrémité de chaque poignée (12).

10. Mallette d'urgence (200) selon la revendication 6, avec une poignée (12) sur seulement l'une des deux coques de mallette (10), un capuchon borgne (42a, 42b) étant prévu sur chacun des coins extérieurs de la coque de mallette (10') sans poignée.

11. Mallette d'urgence (200) selon la revendication 9, dans laquelle un dispositif de centrage d'empilement est réalisé à l'extrémité de la poignée (12) et dans laquelle un dispositif de centrage d'empilement (44a, 44b) est réalisé sur chacun des capuchons borgnes (42a, 42b).

12. Mallette d'urgence (100, 200) selon l'une quelconque des revendications 9 ou 11, dans laquelle les deux dispositifs de centrage d'empilement (30a, 30b, 44a, 44b) d'une coque de mallette (10) sont complémentaires l'un de l'autre.

13. Mallette d'urgence (100, 200) selon l'une quelconque des revendications 1 à 12, dans laquelle chaque coque de mallette (10) fait partie d'une moitié de mallette d'urgence qui est reliée de manière amovible à l'autre moitié de mallette d'urgence.

14. Mallette d'urgence (100, 200) selon l'une quelconque des revendications 1 à 13, dans laquelle chaque coque de mallette (10) dispose d'un couvercle intérieur (26).

15. Mallette d'urgence (100, 200) selon la revendication 14, avec des dispositifs de centrage d'empilement (34a, 34b, 36a, 36b, 38a, 38b, 40a, 40b) sur chaque couvercle intérieur (26).

16. Mallette d'urgence (100, 200) selon l'une quelconque des revendications 1 à 15, avec une paroi au moins partiellement réalisée de manière fluorescente et/ou imprimée de manière fluorescente et/ou collée de manière fluorescente, la fluorescence consistant notamment en une photoluminescence, notamment avec upconversion et/ou downconversion.

17. Mallette d'urgence selon l'une quelconque des revendications 1 à 16, avec une poignée (12) au moins partiellement réalisée de manière fluorescente et/ou imprimée de manière fluorescente et/ou collée de manière fluorescente, la fluorescence consistant notamment en une photoluminescence, notamment avec upconversion et/ou downconversion.

18. Mallette d'urgence avec les caractéristiques de la revendication 16 et de la revendication 17, dans laquelle la poignée (12) est plus fluorescente que la paroi.

19. Mallette d'urgence (100, 200) selon l'une quelconque des revendications 1 à 18, remplie d'au moins un élément parmi :
pansements et matériaux de bandage ;
gants à usage unique ;
ciseaux et pinces de premiers secours ;
masques hygiéniques ;
vêtements hygiéniques ;
douches d'urgence et rince-œil ;
désinfectants et distributeurs associés ;
défibrillateurs et accessoires ;
masques respiratoires.

20. Utilisation d'une mallette avec au moins une coque de mallette (10), une poignée (12) étant réalisée sur la coque de mallette (10), laquelle est agencée avec deux extrémités d'appendice de la poignée (12) respectivement dans une position debout de la mallette d'urgence (100, 200) sur un coin extérieur supérieur de la coque de mallette (10), s'étendant dans une première section verticalement vers le haut et s'étendant dans une deuxième section vers le bord longitudinal intérieur de la coque de la mallette (10) détourné des coins, et une troisième section de la poignée (12) reliant les deux deuxièmes sections s'étendant le long du bord longitudinal intérieur, en tant que mallette d'urgence (100, 200).

21. Utilisation de la mallette d'urgence (100, 200) selon l'une quelconque des revendications 1 à 19 par un praticien médical dans une situation d'urgence médicale ; selon l'utilisation, en partant de la situation où la mallette d'urgence (100, 200) repose sur le sol ou sur un support sur un côté extérieur de la coque de la mallette (10), la troisième section étant saisie à la main par le praticien et tirée vers le praticien.
